(19) Europäisches Patentamt / European Patent Office / Office européen des brevets

(11) **EP 2 399 614 A1**

(12) **EUROPEAN PATENT APPLICATION**

(43) Date of publication:
**28.12.2011 Bulletin 2011/52**

(51) Int Cl.:
*A61L 2/10* (2006.01)   *B01J 19/12* (2006.01)
*C02F 1/00* (2006.01)   *C02F 1/32* (2006.01)

(21) Application number: **10166736.8**

(22) Date of filing: **22.06.2010**

(84) Designated Contracting States:
**AL AT BE BG CH CY CZ DE DK EE ES FI FR GB
GR HR HU IE IS IT LI LT LU LV MC MK MT NL NO
PL PT RO SE SI SK SM TR**
Designated Extension States:
**BA ME RS**

(71) Applicant: **Koninklijke Philips Electronics N.V.
5621 BA Eindhoven (NL)**

(72) Inventors:
• **Mondt, Eva
  5600 AE, Eindhoven (NL)**

• **Taekema, Harko, J
  5600 AE, Eindhoven (NL)**
• **Duineveld, Paulus, C
  5600 AE, Eindhoven (NL)**
• **Mulder, Bernardo, A
  5600 AE, Eindhoven (NL)**

(74) Representative: **Damen, Daniel Martijn
  P.O. Box 220
  5600 AE  Eindhoven (NL)**

(54) **Device for subjecting a fluid to a disinfecting treatment by exposing the fluid to ultraviolet light**

(57)   A device (1) for disinfecting a fluid by exposing the fluid to ultraviolet light comprises the following elements: a fluid treatment area (11) which is adapted to contain fluid and to conduct the fluid through the device (1); means (25, 26) which are adapted to realize a flow of fluid through the fluid treatment area (11); means (20) which are adapted to emit ultraviolet light to the fluid treatment area (11); means (31, 32, 35) which are adapted to monitor operation parameters of a fluid disinfecting treatment which involves a supply of fluid to the fluid treatment area (11) and operation of both the flow realization means (25, 26) and the light emission means (20); and controlling means (30) for determining an optimal flow rate of the fluid through the fluid treatment area (11) and for controlling the operation of elements of the device (1) for actually realizing the optimal flow rate.

Fig. 1

EP 2 399 614 A1

**Description**

FIELD OF THE INVENTION

The present invention relates to a device for subjecting a fluid to a disinfecting treatment by exposing the fluid to ultraviolet light, comprising: a fluid treatment area which is adapted to contain fluid and to conduct the fluid through the device; flow realization means which are adapted to realize a flow of fluid through the fluid treatment area; light emission means which are adapted to emit ultraviolet light to the fluid treatment area; monitoring means which are adapted to monitor operation parameters of a fluid disinfecting treatment which involves a supply of fluid to the fluid treatment area and operation of both the flow realization means and the light emission means; and controlling means which are connected to the monitoring means.

BACKGROUND OF THE INVENTION

A device for subjecting a fluid to a disinfecting treatment by exposing the fluid to ultraviolet (UV) light is well-known. For example, for the microbiological disinfection of drinking water, it is known to use a source/lamp for emitting so-called UV-C radiation, which is ultraviolet light having relatively short waves with a wavelength in an order of 100 to 280 nm. For domestic applications, the UV-C source is usually enclosed in a non-opaque housing, permitting exposure of the fluid to the UV-C radiation while preventing a harmful influence of the radiation on the surroundings. For sufficient germicidal action, a certain dose of ultraviolet radiation is needed, wherein the dose is determined by the irradiance of the source multiplied by a residence time of micro-organisms / pathogens in a fluid treatment area inside the housing. The irradiance of the source is determined by the type of the source and properties of the fluid, especially a capacity of the fluid to transmit ultraviolet light, whereas the residence time is determined by a length of flow paths through the fluid treatment area.

When designing a fluid disinfecting device having an ultraviolet source, it has to be kept in mind that the irradiance of the ultraviolet source decays exponentially with a radial distance to the source. Especially if the fluid to be treated has a low transmittance, a penetration depth of the ultraviolet light into the fluid is limited. Besides, it has to be kept in mind that the ultraviolet output of the source diminishes over time. Especially in the first 100 to 500 burning hours, the UV-C output usually decreases rapidly. Afterwards, the decrease takes place at a much slower rate. In any case, at the end of the lifetime of the source, the UV-C output may be 80-85% of the initial output, or even as low as 60% of the initial output. This is a normal phenomenon in the field of UV-C sources.

Another factor influencing the UV-C output is temperature of the source. For example, in respect of low pressure mercury lamps, an optimal operating temperature is about 30°C. At lower operating temperatures, the UV-C output decreases considerably. At higher operating temperatures, there is also decrease, albeit to a lesser extent.

To ensure proper functioning of a fluid disinfecting device during its entire lifetime, the above-described aspects have to be taken into account. This means that in many practical cases, a fluid disinfecting device is designed considering the worst case operation parameters for a predefined flow rate, which operation parameters involve the lowest expected transmittance of the fluid and the lowest expected output of the ultraviolet source, wherein the latter is actually associated with the end of the lifetime of the source, as explained in the foregoing. As a result, the dose output at the start of the lifetime of the device is significantly higher than required.

In many known devices, variations in ultraviolet output are monitored by a UV-C or visible light sensor. If the ultraviolet output drops below a predefined value, a supply of the fluid to be disinfected is closed in order to avoid an unsafe situation in which the disinfecting process of the fluid is not completed.

Domestic drinking water disinfection devices are usually designed to treat a maximal volume flow of water. This means that a flow controller prevents increase of the flow rate of the water over a maximum value. When flows are lower than the design value, normal treatment is carried out. This could imply that the average dose output is larger than required, i.e. more ultraviolet energy than necessary is used.

Usually, in respect of the variations of UV-C output due to changes in fluid temperature, no measures are taken. In devices using a UV-C sensor as a safety measure, the supply of the fluid is closed in case the ultraviolet output appears to drop below a predetermined value due to temperature decrease. However, a lot of devices use visible light sensors, as that type of sensors is much cheaper. In view of the fact that these sensors are unable to measure the UV-C output directly, unsafe fluid could be supplied.

US 6,972,415 discloses a solution to some of the problems described in the foregoing, and relates to detecting multiple fluid treatment parameters, such as ultraviolet intensity level and fluid flow. In particular, US 6,972,415 discloses a fluid treatment system including an intelligent driver that powers an ultraviolet lamp and has capabilities to receive, process, respond to, and display multiple fluid treatment parameter signals from one or more sensors without the need for additional signal processing and/or control devices. In this context, it is shown that it is possible to have an embodiment in which the power being delivered to the ultraviolet lamp is adjusted as warranted by the intelligent driver's analysis of at least one fluid treatment parameter, such as flow condition.

SUMMARY OF THE INVENTION

It is an object of the present invention to provide a fluid disinfecting device which is completely safe to use, i.e. which will not unexpectedly perform an ineffective disinfecting process, and which is capable of performing the disinfecting process in a most efficient manner, wherein the lifetime of the device, especially the ultraviolet source may be prolonged. The object is achieved by a device comprising a fluid treatment area, flow realization means, light emission means which are adapted to emit ultraviolet light to the fluid treatment area, and monitoring means as defined in the foregoing, as well as controlling means which are connected to both the monitoring means and the flow realization means, which are adapted to calculate an optimal flow rate of the fluid through the fluid treatment area on the basis of actual operation parameters of a fluid disinfecting treatment provided by the monitoring means and a predetermined relation between such operation parameters and an optimal flow rate, and to calculate and realize an associated setting of the flow realization means on the basis of the calculated optimal flow rate and a predetermined relation between a setting of the flow realization means and a flow rate.

On the basis of an application of the controlling means as defined, active control is realized in the device according to the present invention, wherein the flow rate of the fluid through the fluid treatment area can be adjusted. As a result, it is possible to select an optimal operating point for every operation condition. Furthermore, the operating window of the device can be enlarged. For example, when ultraviolet transmission levels are low, less fluid is supplied instead of closing the supply of the fluid, wherein the flow rate is decreased to a level at which a required average dose output is guaranteed. For sake of completeness, it is noted that a practical example of the fluid to be treated by means of the device is water.

Within the scope of the present invention, the definition of the optimal flow rate can be chosen freely. For example, the maximum flow rate at which it is possible to meet a certain dose requirement in the device can be taken as the optimal flow rate. In practice, in order to guarantee that the desired purification of a fluid is achieved, indeed, it is advantageous to use means for controlling a flow of the fluid, such that the velocity distribution in the fluid treatment area, i.e. the flow profile in the fluid treatment area is robust and predictable.

In respect of the dose requirement, it is noted that standards have been developed. For example, according to a standard denoted as NSF/ANSI 55, a minimum dose of 40 mJ/cm$^2$ is required in a Class A, and a minimum dose of 16 mJ/cm$^2$ is required in a Class B.

In contrast to US 6,972,415, the present invention describes a device which does not necessarily control the ultraviolet output on the light emission means, but rather controls the flow rate of fluid in the fluid treatment area. This is more advantageous to the lifetime of the light emitting means, as it is possible for these means to operate throughout the entire lifetime in one and the same operation mode. US 6,972,415 discloses that both the ultraviolet output and the fluid flow are monitored, but the information about these operation parameters is only used to control the light emission means. As a consequence, only the use of the light emission means can be optimized. With the present invention, not only the ultraviolet output can be optimized, but also the flow field, giving more design freedom and allowing for a larger operating window.

In a practical embodiment of the device according to the present invention, the monitoring means are adapted to monitor an ultraviolet output of the light emission means and an ultraviolet transmittance of the fluid. By measuring only the ultraviolet output at a certain spot in the fluid treatment area, as known from US 6,972,415, a distribution of the ultraviolet radiation cannot be determined, and dose control is less accurate.

For a specific lamp type, the combination of an ultraviolet transmission coefficient and a UV-C output of the lamp determines the UV-C distribution in the fluid treatment area. The UV-C output of a lamp is determined by a number of factors, including the input power to the lamp, the efficiency of the ballast/driver, the efficiency of the energy conversion in the plasma or vapor phase (i.e. mercury in low pressure UV lamps), and, if applicable, the efficiency in the coating (phosphor) layer of the glass tube of the lamp, and the operating temperature. In domestic disinfection devices, the UV-C output of the lamp is usually measured at a wall delimiting the fluid treatment area. However, with a single measurement, the UV-C distribution in the fluid treatment area cannot be determined. For example, a certain ultraviolet level at the wall can be achieved either with a low UV-C output of the lamp and a high transmittance of the fluid, or with a high UV-C output of the lamp and a low transmittance of the fluid. Moreover, when a higher ultraviolet level is found at the delimiting wall, this does not automatically imply that the average dose output is sufficient, as this average dose output is also dependent on the distribution of ultraviolet light and the flow field in the fluid treatment area. Thus, for a proper dose output control, it is advantageous to independently monitor both the UV-C output of the lamp and the transmission capacity of the fluid.

The fluid treatment area may be generally cylinder-shaped, and the light emission means may comprise a lamp extending at a central position in the fluid treatment area. In such a case, there are various possibilities for monitoring both an ultraviolet output of the light emission means and an ultraviolet transmittance of the fluid. According to a first possibility, the monitoring means comprise a transmission sensor positioned at an inlet of the fluid treatment area, and a UV-C sensor positioned in a wall delimiting the fluid treatment area. According to a second possibility, the monitoring means comprise a UV-C sensor positioned at a surface of the lamp, and a UV-C sensor positioned in a wall delimiting the fluid treatment area. According to a third possibility, the monitoring means are adapted to measure power supply to the lamp, and comprise a UV-C sensor positioned in one of an inlet of the fluid treatment area and a wall delimiting the fluid treatment area. In the latter case, the ultraviolet output of the lamp is determined by measuring the incoming power to the lamp, and by combining the measured value with known values such as ballast efficiency, efficiency of the conversion of electrical power to UV-C light, and lifetime data of the lamp. The total amount of burning hours can be retrieved from the measurement of the incoming power, or can be derived from a flow counter.

For proper dose control, it is advantageous if also a distribution of the residence time of micro-organisms / pathogens is determined. This can be done by monitoring a flow rate of the fluid through the fluid treatment area. Trajectories followed by fluid elements when passing the light emission means and the associated amounts of ultraviolet radiation received by micro-organisms / pathogens and viruses are determined by the velocity distribution, i.e. the flow profile, which can be found by measuring the flow rate and using a known relation between the flow rate and the flow profile. The optimal flow rate which should be set in the fluid treatment area can then be determined on the basis of a relation between the flow profile and the optimal flow rate. In respect of the relation between the flow rate and the flow profile, it is noted that the shape of the fluid treatment area is an influencing factor. In principle, it is also possible to perform measurements in order to determine the flow profile, but only measuring the flow rate and applying a known relation is very practical.

The monitoring means can comprise a flow sensor, and the flow realization means can comprise a flow controller or a pump, for example. It is advantageous if the flow sensor is arranged outside of the fluid treatment area, at a position upstream or downstream of the fluid treatment area, because in that case, a low cost flow sensor can be used. US 6,972,415 discloses the use of a flow sensor which is positioned in the fluid treatment area, and which is an acoustic flow sensor in order not to disturb the flow. Initial cost of this type of sensor is relatively high. Moreover, entrained gases and/or suspended sediments affect the acoustic signal strength.

As has been explained in the foregoing, it is advantageous to monitor/know the following operation parameters of a fluid disinfecting treatment: the fluid transmission coefficient, the ultraviolet output of the light emission means, the flow rate, and the flow profile. Another parameter which may be monitored is the fluid temperature in the fluid treatment area. This is especially applicable in case the ultraviolet output of the light emission means is dependent on the surrounding temperature. A practical example of such light emission means is a low pressure mercury lamp.

According to the present invention, the controlling means of the fluid disinfecting device serve for determining and setting an optimal flow rate of the fluid in the fluid treatment area. It is also possible that the controlling means are adapted to calculate and realize a setting of the light emission means, using actual operation parameters of a fluid disinfecting treatment provided by the monitoring means and a predetermined relation between such operation parameters and a desired setting of the light emission means. Hence, it is also possible to have a control of the operation of the light emission means as is known from US 6,972,415.

For sake of completeness, it is noted that the monitoring means may be adapted to monitor any combination of operation parameters as mentioned in the foregoing. For example, it may be so that all of the ultraviolet output of the light emitting means, the ultraviolet transmittance of the fluid, the flow rate of the fluid through the fluid treatment area, and the temperature of the fluid in the fluid treatment area are monitored, wherein the flow profile is determined by using the information in respect of the flow rate. In any case, according to the present invention, one or more operation parameters are used to determine an adjustment of the flow rate, which is optimal in the sense that the disinfecting treatment is effective on the one hand, and does not take unnecessary time and energy on the other hand. For example, when the dose output appears to be larger than required, the flow rate can be increased while maintaining the desired disinfecting effect of the treatment of the fluid in the device, and time can be saved. On the other hand, when the dose output appears to be below specifications, which may be due to low transmission levels and/or a drop in ultraviolet output of the light emission means, the flow rate can be decreased such that the treatment of the fluid can still be effective, without a need of closing the supply if the fluid. Furthermore, by controlling the flow rate, it is possible to have a constant operating power of the light emission means, which is beneficial to the lifetime of said means, and it is possible to have an optimal design of the fluid treatment area, which may be smaller than in a conventional situation, as the effectiveness of the treatment of the fluid can be influenced by controlling the flow rate. In any case, in the device according to the present invention, controlling the flow rate is realized by controlling the operation of the flow realization means.

The above-described and other aspects of the present invention will be apparent from and elucidated with reference to the following detailed description of three embodiments of a water disinfecting device.

BRIEF DESCRIPTION OF THE DRAWINGS

The present invention will now be explained in greater detail with reference to the figures, in which equal or similar parts are indicated by the same reference signs, and in which:

> Figure 1 diagrammatically shows a sectional view of components of a water disinfecting device according to a first embodiment of the present invention;
> Figure 2 diagrammatically shows a sectional view of components of a water disinfecting device according to a second embodiment of the present invention;
> Figure 3 diagrammatically shows a sectional view of components of a water disinfecting device according to a third embodiment of the present invention; and
> Figure 4 diagrammatically shows a sectional view of components of a water disinfecting device according to a fourth embodiment of the present invention.

DETAILED DESCRIPTION OF EMBODIMENTS

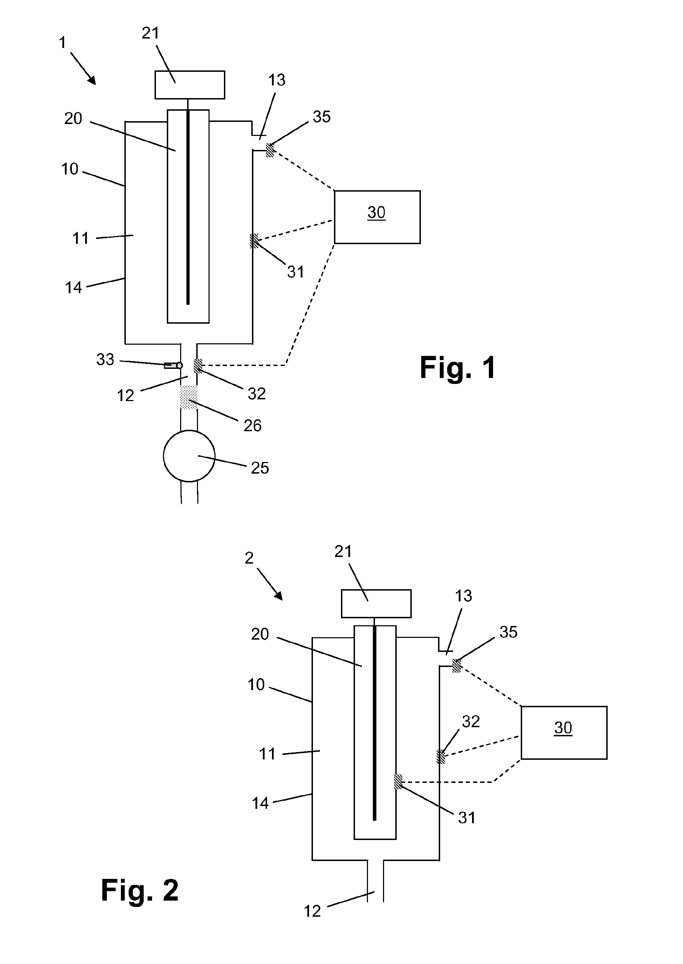

Figures 1-4 show embodiments of a water disinfecting device 1, 2, 3, 4 according to the present invention. In general, the water disinfecting device 1, 2, 3, 4 comprises a reactor 10 having an internal space 11 for containing water and conducting water through the device 1, 2, 3, and a lamp 20 for emitting ultraviolet light, which is arranged inside the reactor 10, at a central position, and which is driven by a driver/ballast 21. For receiving water from water supply means (not shown) the reactor 10 has a water inlet 12, and for discharging water, the reactor 10 has a water outlet 13. When the device 1, 2, 3, 4 is operated, water is made to flow through the reactor 10, under the influence of a pump 25 as diagrammatically shown in figure 1 or other suitable flow realization means, and the lamp 20 is operated. As a result, the water is exposed to the ultraviolet light emitted by the lamp 20 while flowing through the reactor 10, wherein a disinfecting effect on the water is obtained. For sake of completeness, it is noted that practical examples of other flow realization means than a pump 25 include means which are operated on the basis of gravity, and pressurized means for supplying the water, particularly the water main. Also, it is noted that the positioning of the flow realization means may be either upstream or downstream of the reactor 10.

In the following description, it is assumed that the lamp 20 is capable of emitting UV-C light, which is common in the field of water disinfection. For an effective application of the water disinfecting device 1, 2, 3, it is important that all water elements are exposed to a certain dose of the UV-C light, which is found by multiplying the irradiance of the lamp 20 by a residence time of the water elements, especially micro-organisms / pathogens, in the reactor 10. It is noted that the dose is expressed in $(J/m^2)$, the irradiance is expressed in $(W/m2)$, and the residence time is expressed in (s).

The water disinfecting device l, 2, 3, 4 according to the present invention comprises means for monitoring operation parameters of a water disinfecting treatment, as will be explained in the following. Furthermore, controlling means 30 such as a microcontroller are provided for processing signals derived from the monitoring means, and controlling the operation of various elements of the device 1, 2, 3, 4 on the basis of an outcome of the said signal processing. As is usual with controlling means 30, they are programmed in a certain way, wherein algorithms are followed during their operation, on the basis of which output can be obtained on the basis of the input. In the figures, the controlling means 30 are depicted as a box, wherein signal connections between the controlling means 30 and various monitoring elements are depicted by means of dashed lines.

In the first place, the controlling means 30 are adapted to determine a distribution of ultraviolet light in the reactor 10. To that end, an ultraviolet output of the lamp 20 and a capacity of the water to transmit the ultraviolet light are monitored independently. For this purpose, two sensors 31, 32 can be used, wherein one sensor 31 measures the ultraviolet output of the lamp 20, and wherein the other sensor 32 measures the ultraviolet transmittance of the water. Another option is to determine the ultraviolet output on the basis of incoming power and lifetime data of the lamp 20, in which case a relation between those two parameters and the ultraviolet output should be stored in the controlling means 30.

In the first embodiment of the water disinfecting device 1 as shown in figure 1, the sensor 31 for measuring the ultraviolet output of the lamp 20 comprises a UV-C sensor 31 positioned in a wall 14 of the reactor 10, and the sensor 32 for measuring the ultraviolet transmittance of the water comprises a sensor 32 positioned at the water inlet 12 of the reactor 10. A suitable light source 33 for emitting light to be detected by the sensor 32 at the water inlet 12 of the reactor 10 is provided as well.

The sensor 32 for measuring the ultraviolet transmittance of the water may be a UV-C sensor, wherein the light source 33 is a UV-C light source. A cheaper option is to use a light emitting diode (LED) and a photodiode or cadmium sulfide (CdS) sensor in the visible light range. This cheaper option is especially applicable if a transmission change in the visible light range correlates to transmission in the UV-C range, as is the case for low pressure mercury lamps, for example.

On the basis of the measured transmittance at the water inlet 12 of the reactor 10, and the output of the sensor 31 positioned at the reactor wall 14, the UV-C output of the lamp 20 can be determined.

In the second embodiment of the water disinfecting device 2 as shown in figure 2, a separation of a measurement of the ultraviolet output and the ultraviolet transmittance is realized by using two UV-C sensors 31, 32 at different locations inside the reactor 10. One sensor 31 is located at a surface of the lamp 20, and serves for measuring the output. The other sensor 32 is located at the reactor wall 14, and together with the measurement value of the first sensor 31, the transmittance can be determined. If the output in the UV-C range and in the visible light range correlate, application of a visible light sensor is sufficient to determine the amount of ultraviolet power emitted by the lamp 20, provided that the sensor is resistant to UV-C light.

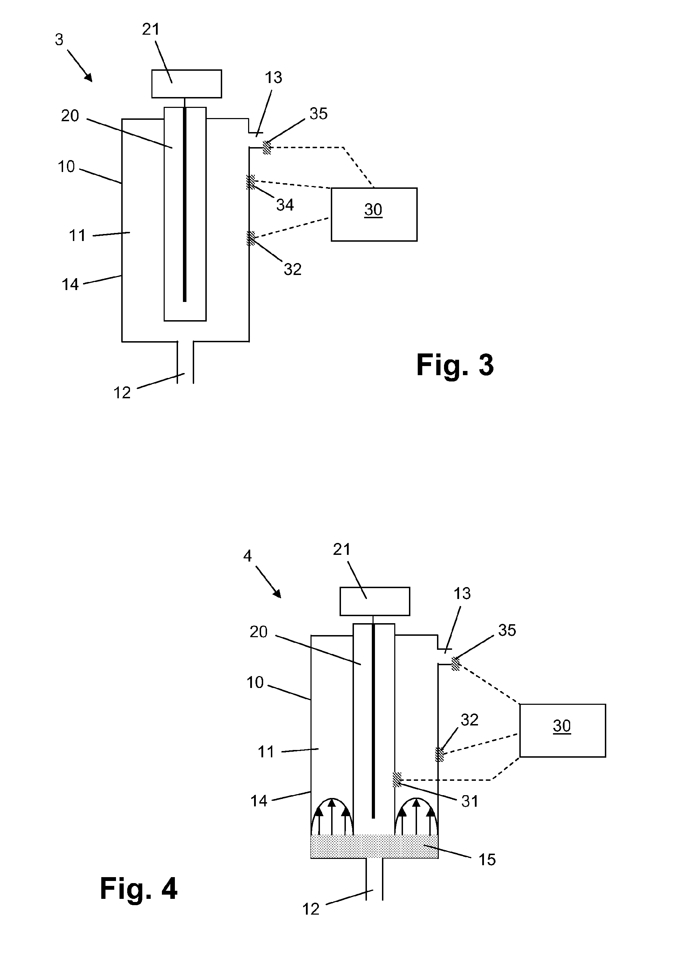

In the third embodiment of the water disinfecting device 3 as shown in figure 3, the ultraviolet output is determined by measuring the incoming power/current to the lamp 20. Known values of the ballast efficiency, efficiency of conversion of electrical power to UV-C light, and lifetime data of the lamp 20, are used to determine the ultraviolet output on the basis of the measured incoming power/current. The amount of burning hours can be retrieved from the measurement of the incoming power, or can be derived from a flow counter. Furthermore, a combination of a sensor 32 and a light source 33 positioned at the water inlet 12 of the reactor 10, as known from the first embodiment, or a UV-C sensor 32 located in the reactor wall 14 allows for measurement of the water transmittance.

If the lamp 20 has a light output which is dependent on temperature, it is advantageous to monitor the fluid temperature inside the reactor 10. In that case, a temperature sensor 34 may be used and arranged in the reactor wall 14, as shown in figure 3. By using known relations between ultraviolet output and temperature, the electrical power input to the lamp 20 can be controlled such that the dose output is sufficient.

The most common UV-C sensors are photodiodes. A photodiode is a type of photo detector capable of converting light into either current or voltage, depending upon the mode of operation. Another way to determine the amount of UV-C light is by using a temperature sensor. This sensor should be placed behind a glass plate, allowing only transmission of UV-C light (no visible and infrared light). As the temperature of the incoming light is wavelength dependent, the ultraviolet output can be correlated.

The UV-C sensors do not need necessarily to be attached to a wall or the like, as it is also possible to use floating sensors. However, floating sensors are not preferred, because such sensors will distort the flow profile and cause shadowing, i.e. cause part of the fluid to be blocked from the UV-C light.

In the second place, the controlling means 30 are adapted to determine a flow behavior of the water in the reactor 10, especially a flow rate and a flow profile. The flow rate is determined by characteristics of the supply of the water, for example, input pressure. The flow profile is mainly determined by flow rate and design of the reactor 10.

One way of controlling the flow rate through the reactor 10 involves using a flow sensor 35 in combination with a flow controller 26 as diagrammatically shown in figure 1, which can be arranged either upstream or downstream of the reactor 10, and a regulator. Another way of controlling the flow rate through the reactor 10 involves using a flow sensor 35 in combination with a pump 25 and a regulator.

Preferably, a low cost sensor 35 is used for measuring the flow rate, wherein the sensor 35 is arranged outside of the reactor 10, either upstream or downstream of the reactor. A common low cost flow sensor is a turbine-like flow meter. The vanes on the turbine start spinning under the influence of the flowing water. The rate of the spin is measured and correlated to a fluid velocity. Another way of measuring the flow rate is by use of the so-called calorimetric principle. The calorimetric principle for fluid flow measurement is based on the use of two temperature sensors which are in close contact with the fluid, yet thermally insulated from each other. One of the two temperature sensors is constantly heated, and the cooling effect of the flowing fluid is used to monitor the flow rate. In a stationary fluid condition, i.e. in a condition in which there is no fluid flow, there is a constant temperature difference between the two temperature sensors. When the fluid flow increases, heat energy is drawn from the heated sensor and the temperature difference between the sensors is reduced, wherein the reduction is proportional to the flow rate of the fluid. Also, magnetic flow sensors could be used for measuring the flow rate. Although they are more expensive than turbine flow meters, they have the advantage that they do not obstruct the flow and do not have moving parts.

For controlling the flow rate, a valve 26 which can be set in various positions may be used. Hence, it is not intended to apply a valve for starting and stopping the flow. Instead, it is advantageous to have a valve 26 by means of which the flow rate can be accurately regulated. Examples of suitable valves comprise closing-down valves like piston valves, needle valves or globe valves, and rotary valves like ball valves. Closing-down valves are suitable for the task of setting a flow rate because of a directly proportional relationship between the size of a seat opening of the valve and the travel of a closure member of the valve. Rotary valves also offer good throttling control, but normally only over a restricted valve opening range. Control of the valve position can be achieved by electrical actuation, for example, by using a step motor. Another option is to use a pin in an electrical wiring to change position of the pin and hence change the open area of an inlet of the valve 26.

Another way to regulate the flow is by use of a pump 25. Suitable pump types are piston pumps and centrifugal pumps. Piston pumps are positive displacement pumps which can be designed to pump at practically constant flow rates (averaged over time) against a wide range of discharge pressures. The piston may be driven by an electric motor to supply water at adjustable flow rates. A centrifugal pump uses a rotating impeller to increase the pressure of a fluid. When regular variations in flow rate occur, centrifugal pumps are favored.

Accurately setting the flow rate is achieved by coupling of a signal provided by the flow sensor 35 with the operation of the flow controller/pump 25, 26. The signal from the flow sensor 35 is transferred to a suitable regulation unit (not shown), which may be electronic, mechanical, or hydraulic. This regulation unit, which may be a PID regulator, for example, drives an electrical actuator of the valve 26 or the pump 25. When a valve 26 is used to control the flow, an inherent valve flow characteristic is an important design parameter. Such characteristic describes a relation between a valve opening and the flow rate. For accurate flow control over a wide range, a (near) linear relation, which determines that the flow capacity increases linearly with the valve travel, is preferred. For example, a quick opening characteristic or a hyperbolic characteristic, which is characterized by large changes in flow for very small changes in valve area, is not very well suitable for accurate flow control.

Advantageously, for realizing an optimal water disinfecting treatment, a flow profile, i.e. a distribution of the flow inside the reactor 10 should be known, apart from the flow rate, to be able to actively control the dose output. The reason is that actual trajectories traveled by the water elements together with the ultraviolet distribution determine the total dose output. In most reactor designs, the flow profile changes when the flow rate is changed. Therefore a robust, easy to predict reactor design is preferred.

The flow profile can be determined from analytical relations (parabolic and plug flow in the laminar region), from numerical studies (Computational Fluid Dynamics), or from measurements. Robust reactor design is preferred in order to be able to accurately determine/predict the flow profile in the reactor 10 as a function of flow rate, otherwise it is likely to happen that small distortions at the inlet 12 of the reactor 10 lead to large, and possibly even unpredictable, changes of the velocity profile in the reactor 10. One way of realizing a robust and predictable velocity profile in the reactor 10 is having a flow straightener 15 such as a foam element, which is placed near the inlet 12 in order to create a laminar parabolic velocity profile, as diagrammatically shown in figure 4.

The measured flow rate and the known fluid velocity distribution inside the reactor 10, together with the measured ultraviolet output of the lamp 20 and the measured water transmittance, allows for optimum adjustment of the flow rate and/or the lamp output.

The controlling means 30 may use dedicated software to read out the various sensors 31, 32, 34, 35 (ultraviolet output, ultraviolet transmittance of the water, temperature, flow rate), to transform the measurement data into the required information, and to control the flow controller/pump 25, 26 and possibly also the driver 21 for the lamp 20 to respectively set the required volume flow rate through the reactor 10 and possibly also the ultraviolet output of the lamp 20.

By using active dose control, several benefits can be achieved. First, when the ultraviolet output of the lamp 20 is controlled, power input to the lamp 20 can be reduced in situations in which the fluid has a large ultraviolet transmittance and/or in which the flow rate drops, the power input to the lamp 20 can be reduced. As a result, the energy consumption of the lamp 20 is reduced.

Active control of the fluid behavior inside the reactor 10 gives extra benefits. As indicated before, the operating window of the device 1, 2, 3, 4 can be enlarged. For example, when the dose output is larger than required, the flow rate through the device 1, 2, 3, 4 can be increased. Tests which have been performed in the context of the present invention have shown that a flow output can be increased with at least 50% when transmission levels of the fluid increase from 70% to 90-95%. Hence, flow rates can be increased significantly when proper dose control is performed, whereby it is possible to fulfill customer needs. On the other hand, in case of a dose output below specifications, the flow rate can be decreased such that the required output is achieved, instead of the conventional procedure of closing the supply of fluid.

Another advantage of active flow control is the possibility to operate the UV-C source 20 at a constant level. Instead of adjusting the input power to the source 20, the flow rate through the device 1, 2, 3, 4 is controlled such that the required dose output is achieved. This is beneficial for the life time of the UV-C source 20, as it does not need to cope with different operating powers.

Furthermore, active flow control allows for optimization of the design of the reactor 10. Conventionally, the reactor design is usually optimized for a certain ultraviolet output characteristic and certain qualification standards. For domestic applications, this means that optimization is done for a fluid transmission of 70%. However, in most operating conditions, transmission levels will be much larger (> 85%) due to the presence of a prefiltering system, for example, an active carbon block. As a result, the conventional device is in most practical situations over specified, meaning that the average residence time of the fluid inside the reactor 10 is larger than required. By active control of the flow rate and measurement of the UV-C level, it becomes possible to decrease the reactor volume. In case the water transmission is lower than designed for, the active flow control allows reducing the flow rate through the device 1, 2, 3, 4.

In the following, an example of how the present invention can be applied in practice will be described. In this example, both the ultraviolet output of a lamp 20 and the flow profile of water flowing around the lamp 20 are used for accurately controlling a dose output in a device 1, 2, 3, 4 for disinfecting drinking water.

In general, the dose that is generated in a device using ultraviolet radiation for disinfecting water or another fluid depends on both a spacial velocity profile $v_Q$ (r, $\varphi$, z) and an irradiance I. The velocity profile $v_Q$ (r, $\varphi$, z) integrated over the flow through cross-section of the reactor 10 gives a flow rate Q. The flow rate Q can be imposed by a pump 25. The velocity profile v_Q (r, φ, z) changes with the flow rate Q. The irradiance I depends on the type of lamp 20 and driver 21 used, the turbidity of the fluid γ, the time over lifetime of the lamp 20 τ and the temperature of the surrounding fluid T.

Infinitesimal material fluid volume enters the reactor 10 at r = r_0, φ = φ_0, z = 0. The total dose D (r, (φ) received by any material fluid volume should at least be more than a minimally required dose, as follows, wherein ds stands for a path that is traveled by the micro-organisms, i.e. the path from the inlet 12 to the outlet 13, which path is determined by the dimensions of the reactor 10, and possibly also the flow rate Q:

$$D(r, \varphi) = \int\limits_{entrance\,reactor}^{exit\,reactor} I_{(\tau, T, \gamma)}(r, \varphi, z) \cdot \frac{\overrightarrow{ds}}{v_Q(r, \varphi, z)} \geq D_{\min imal\,required}$$

Hence, in this example, the following parameters should be known: the spacial velocity profile v_Q (r, φ, z), the flow rate Q, the ultraviolet output at a surface of the lamp 20, the turbidity of the fluid γ, and, optionally, the time over lifetime of the lamp 20 τ and/or the temperature of the fluid T.

The velocity profile v_Q (r, φ, z) can be set by adjusting the flow rate Q. Also, the impact of a change in the flow rate Q on a change of the velocity profile v_Q (r, φ, z) should be known. The relation between the velocity profile v_Q (r, φ, z) and the flow rate Q is determined by specifics of the reactor 10. In most reactor designs, the velocity profile v_Q (r, φ, z) changes when the flow rate Q is changed. In some cases, this change is predictable, and in other cases, it is not. When this change is predictable, no large velocity differences and recirculation zones are present in the reactor 10, and a very smooth flow pattern exists.

Using a reactor 10 in which a robust and predictable flow pattern exists is very useful when it comes to applying dose control. The reason is that in respect of such a reactor 10, the relation between the flow rate Q and the spacial velocity profile v_Q (r, φ, z) can be described by a simple equation, whereas in the case of a fluctuating velocity profile v_Q (r, φ, z), the velocity distribution needs to be determined for every flow rate Q, and stored in a memory unit. Moreover, in the case of a robust reactor design, changes in inlet conditions lead to a predictable change of the velocity profile v_Q (r, φ, z) in the reactor 10, whereas for a reactor 10 which is less stable unknown effects may occur, which lead to a different velocity profile v_Q (r, φ, z) than expected, and which may have a negative effect on the dose output D.

The irradiance I can be controlled and/or known by taking proper technical measures known per se. The minimum required dose D is determined by legislation. For devices using ultraviolet radiation for treating fluids, a standard denoted as NSF/ANSI 55, Class A or Class B, applies.

All data as mentioned, particularly the spacial velocity profile v_Q (r, φ, z), the flow rate Q, the irradiance I, the turbidity of the fluid γ, the time over lifetime of the lamp 20 τ and the temperature of the surrounding fluid T can be stored in a memory unit. From these data, an optimal value for the flow rate Q and the irradiance I can be determined using a suitable algorithm, which may comprise the elements listed in the following.

1) Start control cycle
2a) Receive signal from UV sensors 31, 32, and determine irradiance I at lamp surface and turbidity γ from input signals and data stored in memory unit
2b) Receive signal from flow sensor(s) 35, and determine velocity profile v_Q (r, φ, z) from input signal and relation between flow rate Q and velocity profile v_Q (r, φ, z) stored in memory unit
2c) Receive signal from temperature sensor, and determine irradiance I at lamp surface from input signal and relation between temperature of fluid T and irradiance I stored in memory unit (optional, depending on choice of how to monitor irradiance I at lamp surface)
2d) Receive signal from power supply to lamp 20, and determine irradiance I at lamp surface from input signal and relation between power and irradiance I stored in memory unit (optional, depending on choice of how to monitor irradiance I at lamp surface)
2e) Receive signal from internal clock, and determine irradiance I at lamp surface from input signal and relation between time over lifetime of lamp 20 τ and irradiance I stored in memory unit (optional, depending on choice of how to monitor irradiance I at lamp surface)
3) Calculate actual dose output D_actual on the basis of the equation as given in the foregoing
4a) If actual dose output D_actual < (required dose output D_required + x%), wherein x is a safety margin: decrease flow rate Q, decrease valve opening or pump speed
4b) If actual dose output D_actual > (required dose output D_required + y%), wherein y is a safety margin that is larger than x: increase flow rate Q, increase valve opening or pump speed
4c) If (required dose output D_required + y%) ≥ actual dose output D_actual ≥ (required dose output D_required + x%): no action required

In this example, the flow rate Q is adjusted. Additionally or alternatively, the power supply to the lamp 20 can be adjusted. An intended effect of decreasing the flow rate Q can also be achieved by increasing the power at which the lamp 20 is operated, and an intended effect of increasing the flow rate Q can also be achieved by decreasing the power at which the lamp 20 is operated. In any case, the example shows that there are practical ways of setting an optimal dose value. An optimal dose value may be a value at which at least one of the following is true:

- the lifetime of the lamp 20 is increased;
- the power consumed by the lamp 20 is decreased;
- the operating window of the device 1, 2, 3, 4 is enlarged, i.e. the range of flow rates Q is enlarged; and
- the reactor design volume is optimized.

It will be clear to a person skilled in the art that the scope of the present invention is not limited to the examples discussed in the foregoing, but that several amendments and modifications thereof are possible without deviating from the scope of the present invention as defined in the attached claims. While the present invention has been illustrated and described in detail in the figures and the description, such illustration and description are to be considered illustrative or exemplary only, and not restrictive. The present invention is not limited to the disclosed embodiments.

Variations to the disclosed embodiments can be understood and effected by a person skilled in the art in practicing the claimed invention, from a study of the figures, the description and the attached claims. In the claims, the word "comprising" does not exclude other steps or elements, and the indefinite article "a" or "an" does not exclude a plurality. The mere fact that certain measures are recited in mutually different dependent claims does not indicate that a combination of these measures cannot be used to advantage. Any reference signs in the claims should not be construed as limiting the scope of the present invention.

It is noted that in the context of this description, the terms "ultraviolet light" and "ultraviolet radiation" are used to indicate one and the same phenomenon, namely light waves having a wavelength in the ultraviolet region.

In the embodiments of the device 1, 2, 3, 4 as shown in the figures, a flow of a fluid is substantially parallel to a lamp 20 having an elongated shape, i.e. substantially parallel to a longitudinal axis of the lamp 20. That does not alter the fact that within the scope of the present invention, the flow may also be perpendicular to the longitudinal axis of the lamp 20.

The light emission means 20 of the device 1, 2, 3, 4 may comprise a single lamp 20, as is the case in the shown examples, but it is also possible for the light emission means 20 to comprise multiple lamps.

The present invention can be summarized as follows. A device 1, 2, 3, 4 for subjecting a fluid to a disinfecting treatment by exposing the fluid to ultraviolet light comprises the following elements: a fluid treatment area 11 which is adapted to contain fluid and to conduct the fluid through the device 1, 2, 3, 4; flow realization means 25 which are adapted to realize a flow of fluid through the fluid treatment area 11, for example, a pump 25; light emission means 20 which are adapted to emit ultraviolet light to the fluid treatment area 11, for example, a UV-C lamp 20; monitoring means 31, 32, 34, 35 which are adapted to monitor operation parameters of a fluid disinfecting treatment which involves a supply of fluid to the fluid treatment area 11 and operation of both the flow realization means 25 and the light emission means 20, for example, a UV-C sensor 31, 32 and/or a flow sensor 35; and controlling means 30 which are connected to both the monitoring means 31, 32, 34, 35 and the flow realization means 25, and which are adapted to determine an optimal flow rate of the fluid through the fluid treatment area 11 and to control the operation of elements of the device 1, 2, 3, 4, especially the flow realization means 25, for actually realizing the optimal flow rate.

**Claims**

**1.** Device (1, 2, 3, 4) for subjecting a fluid to a disinfecting treatment by exposing the fluid to ultraviolet light, comprising:

- a fluid treatment area (11) which is adapted to contain fluid and to conduct the fluid through the device (1, 2, 3, 4);
- flow realization means (25, 26) which are adapted to realize a flow of fluid through the fluid treatment area (11);
- light emission means (20) which are adapted to emit ultraviolet light to the fluid treatment area (11);
- monitoring means (31, 32, 34, 35) which are adapted to monitor operation parameters of a fluid disinfecting treatment which involves a supply of fluid to the fluid treatment area (11) and operation of both the flow realization means (25, 26) and the light emission means (20);
- controlling means (30) which are connected to both the monitoring means (31, 32, 34, 35) and the flow realization means (25, 26), which are adapted to calculate an optimal flow rate of the fluid through the fluid treatment area (11) on the basis of actual operation parameters of a fluid disinfecting treatment provided by the monitoring means (31, 32, 34, 35) and a predetermined relation between such operation parameters and an optimal flow rate, and to calculate and realize an associated setting of the flow realization means (25, 26) on the basis of the calculated optimal flow rate and a predetermined relation between a setting of the flow realization means (25, 26) and a flow rate.

2. Device (1, 2, 3, 4) according to claim 1, wherein the monitoring means (31, 32) are adapted to monitor an ultraviolet output of the light emission means (20) and an ultraviolet transmittance of the fluid.

3. Device (1) according to claim 2, wherein the fluid treatment area (11) is generally cylinder-shaped, wherein the light emission means comprise a lamp (20) extending at a central position in the fluid treatment area (11), and wherein the monitoring means comprise a transmission sensor (32) positioned at an inlet (12) of the fluid treatment area (11), and a UV-C sensor (31) positioned in a wall (14) delimiting the fluid treatment area (11).

4. Device (2, 4) according to claim 2, wherein the fluid treatment area (11) is generally cylinder-shaped, wherein the light emission means comprise a lamp (20) extending at a central position in the fluid treatment area (11), and wherein the monitoring means comprise a UV-sensor (31) positioned at a surface of the lamp (20), and a UV-C sensor (32) positioned in a wall (14) delimiting the fluid treatment area (11).

5. Device (3) according to claim 2, wherein the fluid treatment area (11) is generally cylinder-shaped, wherein the light emission means comprise a lamp (20) extending at a central position in the fluid treatment area (11), and wherein the monitoring means are adapted to measure power supply to the lamp (20), and comprise a UV-C sensor (32) positioned in one of an inlet (12) of the fluid treatment area (11) and a wall (14) delimiting the fluid treatment area (11).

6. Device (1, 2, 3, 4) according to claim 1, wherein the monitoring means (35) are adapted to monitor a flow rate of the fluid through the fluid treatment area (11).

7. Device (1, 2, 3, 4) according to claim 6, wherein the predetermined relation between the operation parameters of a fluid disinfecting treatment and an optimal flow rate of the fluid through the fluid treatment area (11) is based on both a relation between the flow rate of the fluid and a flow profile of the fluid and a relation between the flow profile of the fluid and an optimal flow rate.

8. Device (1, 2, 3, 4) according to claim 1, wherein the optimal flow rate is defined as a maximum flow rate at which it is possible to meet a predetermined dose requirement, i.e. a requirement pertaining to an irradiance of the light emission means multiplied by a residence time of fluid element in the fluid treatment area.

9. Device (1, 2, 3, 4) according to claim 8, wherein the predetermined dose requirement involves a minimum dose of 16 mJ/cm$^2$, preferably 40 mJ/cm$^2$.

10. Device (1, 2, 3, 4) according to claim 1, wherein the monitoring means comprise a flow sensor (35), and wherein the flow realization means (25, 26) comprise a flow controller (26).

11. Device (1, 2, 3, 4) according to claim 1, wherein the monitoring means comprise a flow sensor (35), and wherein the flow realization means (25, 26) comprise a pump (25).

12. Device (1, 2, 3, 4) according to claim 1, wherein the monitoring means comprise a flow sensor (35) which is positioned outside of the fluid treatment area (11), at one of a position upstream of the fluid treatment area (11) and a position downstream of the fluid treatment area (11).

13. Device (3) according to claim 1, wherein the monitoring means (34) are adapted to monitor a temperature of the fluid in the fluid treatment area (11).

14. Device (1, 2, 3, 4) according to claim 1, wherein the controlling means (30) are furthermore adapted to calculate and realize a setting of the light emission means (20) on the basis of actual operation parameters of a fluid disinfecting treatment provided by the monitoring means (31, 32, 34, 35) and a predetermined relation between such operation parameters and a desired setting of the light emission means (20).

Fig. 1

Fig. 2

**Fig. 3**

**Fig. 4**

Europäisches
Patentamt

European
Patent Office

Office européen
des brevets

## EUROPEAN SEARCH REPORT

Application Number

EP 10 16 6736

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 4 471 225 A (HILLMAN LEON [US]) 11 September 1984 (1984-09-11) * the whole document * | 1-9, 11-14 | INV. A61L2/10 B01J19/12 C02F1/00 |
| X | WO 01/29535 A1 (LIGHTSTREAM TECHNOLOGIES INC [US]; LANTIS ROBERT M [US]) 26 April 2001 (2001-04-26) * page 12, line 15 - page 21, line 21; figure 3 * | 1,2,4-14 | C02F1/32 |
| X | WO 2007/031041 A1 (SCHNEIDER STEPHAN [DE]; HEYER UWE [DE]) 22 March 2007 (2007-03-22) * page 1 - page 2 * * page 6, paragraph 5 * * page 7, paragraph 4 * * claims 1,6; figures 1-2 * | 1-6,8,10 | |
| X | WO 00/20045 A1 (COMMON SERVICES AGENCY [GB]; IATROS LTD [GB]; GUNN ANDREW [GB]; CAMERO) 13 April 2000 (2000-04-13) * page 26, line 31 - page 30, line 2 * * page 32, line 15 - page 34, line 10; figures 1,8 * | 1,6-13 | |
| X | US 4 302 677 A (ALBERTSSON NILS L ET AL) 24 November 1981 (1981-11-24) * claim 1 * | 1,2,4,8, 13 | TECHNICAL FIELDS SEARCHED (IPC) A61L B01J C02F |
| X | WO 00/68152 A1 (PINEL MILTON PAUL [CA]; DOUGLAS NOEL K [CA]) 16 November 2000 (2000-11-16) * page 6, line 12 - page 7, line 18; figure 2 * | 1,2,4,8, 13 | |
| A | US 6 972 415 B2 (SCHALBLE UWE D [CA] ET AL SCHAIBLE UWE D [CA] ET AL) 6 December 2005 (2005-12-06) * the whole document * | 1-14 | |

The present search report has been drawn up for all claims

| Place of search | Date of completion of the search | Examiner |
|---|---|---|
| Munich | 24 August 2010 | Marti, Pedro |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 10 16 6736

This annex lists the patent family members relating to the patent documents cited in the above-mentioned European search report.
The members are as contained in the European Patent Office EDP file on
The European Patent Office is in no way liable for these particulars which are merely given for the purpose of information.

24-08-2010

| Patent document cited in search report | | Publication date | Patent family member(s) | | Publication date |
|---|---|---|---|---|---|
| US 4471225 | A | 11-09-1984 | NONE | | |
| WO 0129535 | A1 | 26-04-2001 | AR | 026212 A1 | 29-01-2003 |
| | | | AU | 772315 B2 | 22-04-2004 |
| | | | AU | 1094901 A | 30-04-2001 |
| | | | CA | 2395801 A1 | 26-04-2001 |
| | | | CN | 1413297 A | 23-04-2003 |
| | | | CZ | 20022214 A3 | 12-03-2003 |
| | | | EP | 1242806 A1 | 25-09-2002 |
| | | | HU | 0203846 A2 | 28-07-2003 |
| | | | PL | 356670 A1 | 28-06-2004 |
| | | | TW | 512228 B | 01-12-2002 |
| | | | US | 6264836 B1 | 24-07-2001 |
| WO 2007031041 | A1 | 22-03-2007 | DE | 112005003760 A5 | 28-08-2008 |
| WO 0020045 | A1 | 13-04-2000 | AT | 361104 T | 15-05-2007 |
| | | | AU | 6099599 A | 26-04-2000 |
| | | | CA | 2345423 A1 | 13-04-2000 |
| | | | EP | 1117445 A1 | 25-07-2001 |
| | | | JP | 2002526169 T | 20-08-2002 |
| | | | TW | 436303 B | 28-05-2001 |
| | | | TW | 474828 B | 01-02-2002 |
| | | | US | 6586172 B1 | 01-07-2003 |
| US 4302677 | A | 24-11-1981 | DE | 3011817 A1 | 09-10-1980 |
| | | | ES | 8107127 A1 | 16-12-1981 |
| | | | FR | 2452289 A1 | 24-10-1980 |
| | | | IT | 1143189 B | 22-10-1986 |
| | | | SE | 8000062 A | 28-09-1980 |
| WO 0068152 | A1 | 16-11-2000 | AU | 4530600 A | 21-11-2000 |
| | | | CA | 2336524 A1 | 16-11-2000 |
| US 6972415 | B2 | 06-12-2005 | US | 2004061069 A1 | 01-04-2004 |

EPO FORM P0459

For more details about this annex : see Official Journal of the European Patent Office, No. 12/82

**REFERENCES CITED IN THE DESCRIPTION**

**Patent documents cited in the description**

- US 6972415 B